# FASCICULE DE BREVET EUROPEEN

(11) **EP 0 358 550 B1**
(45) Date de publication et mention de la délivrance du brevet: **30.03.1994**
(21) Numéro de dépôt: 89402384.5
(22) Date de dépôt: 01.09.1989
(51) Int. Cl.: C07C 211/53, C07C 215/18, C07C 211/52, C07C 215/16, A61K 7/13

(54) **Procédé de préparation d'alfa, omega-diamines aliphatiques, composés nouveaux et leur utilisation en teinture capillaire**
Verfahren zur Herstellung von alifatischen alpha-omega-Diaminen, neue Verbindungen und deren Verwendung für die Haarfärbung
Process for the preparation of aliphatic alpha-, omega-diamines, new compounds and their use in hair dying

(30) Priorité: 06.09.1988 FR 8811634
(43) Date de publication de la demande: 14.03.1990
(73) Titulaire: L'OREAL, 75008 Paris (FR)
(72) Inventeur: Junino, Alex, F-93180 Livry-Gargan (FR); Genet, Alain, F-93600 Aulnay-sous-Bois (FR); Lang, Gérard, F-95210 Saint-Gratien (FR)
(74) Mandataire: Casalonga, Axel

(56) Documents cités:
- DE-A- 1 939 062
- DE-A- 2 650 764
- FR-A- 2 443 705

## Description

La présente invention est relative à un nouveau procédé de préparation d'α, ω-diamines aliphatiques N,N′-tétrasubsituées, aux nouveaux composés constitués par le N,N′-bis(β-hydroxyéthyl)N,N′-bis (4′-aminophényl)1,3-diaminopropane-2-ol et à la N,N′-bis(éthyl)N,N′-bis(4′-amino 3′-méthylphényl)éthylène diamine, leurs sels d'addition avec les acides et leur utilisation en teinture capillaire d'oxydation.

On a déjà décrit dans le brevet français 2.016.123 et le brevet US-3.694.138 un procédé de préparation de N,N′-diphénylalkylènediamines, dont les cycles phényle sont substitués en para par un groupement OH ou amino, ce groupement amino étant éventuellement substitué par un radical alkyle et les groupements amines pouvant eux-mêmes être substitués par un groupement alkyle, hydroxyalkyle ou aminoalkyle.

Le procédé de préparation de ces composés consiste essentiellement à effectuer, dans une première étape, une réaction de tosylation de la p-acétamino aniline pour obtenir la N-acétyl N′-tosylparaphénylène diamine, à faire suivre, dans une seconde étape, par une condensation d'un dihalogénohydrocarbure sur un sel alcalin ou alcalino-terreux de la N-acétyl N′-tosyl paraphénylènediamine, à procéder, dans une troisième étape, à une hydrolyse sélective des groupements tosyle de la N,N′-bis-tosyl N,N′-bis-(4′-acétamidophényl) alkylènediamine obtenue au cours de la deuxième étape. Cette hydrolyse s'effectue à l'aide d'acide sulfurique à froid et conduit à la N,N′-bis-(4′-acétamidophényl) alkylènediamine.

La quatrième étape de ce procédé antérieur est une alkylation ou une hydroxyalkylation ou amino alkylation des fonctions amines secondaires.

La cinquième étape consiste enfin à procéder à l'hydrolyse de la fonction acétamido par action de l'acide chlorhydrique à chaud.

Ce procédé présente l'inconvénient d'être long et coûteux et difficile à mettre en oeuvre industriellement.

La demanderesse a découvert, ce qui fait l'objet de la présente invention, un nouveau procédé de préparation beaucoup plus simple et rapide et mettant en oeuvre des matières premières couramment utilisées.

Ce procédé est représenté par le schéma réactionnel A.

### SCHEMA REACTIONNEL A

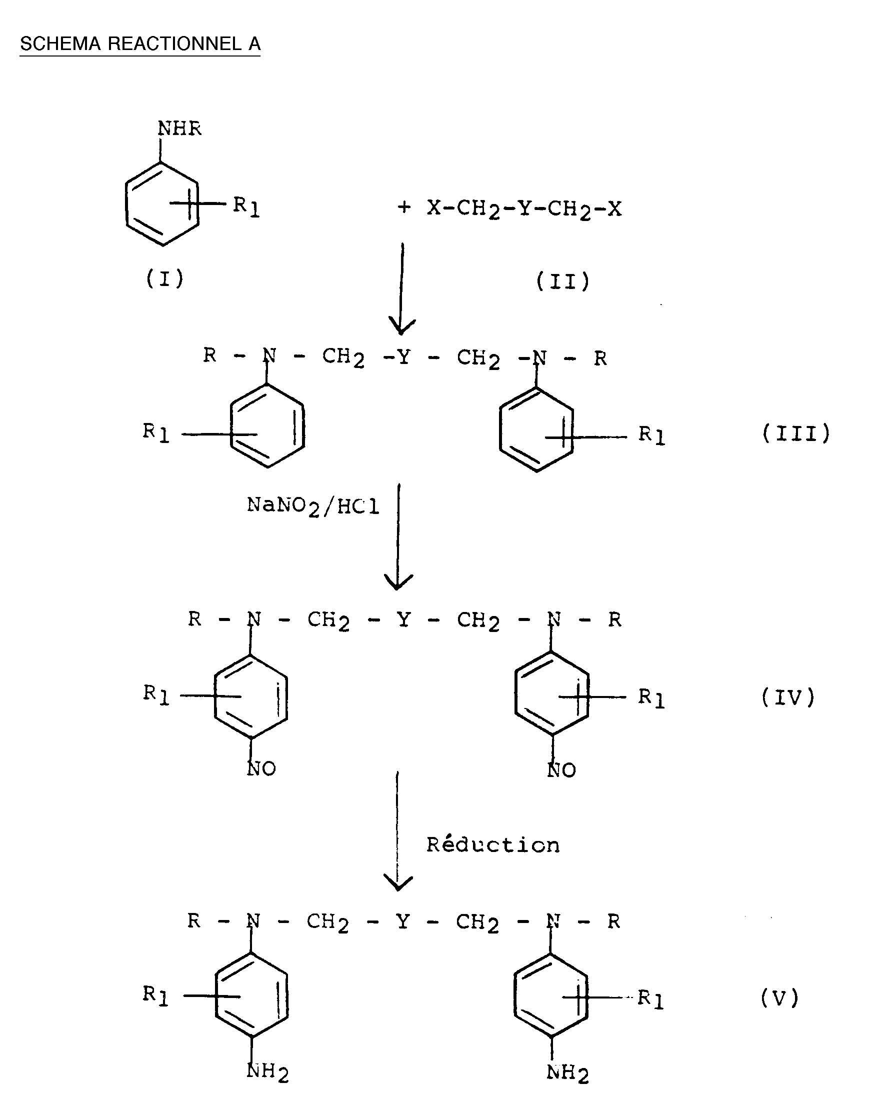

Dans les différentes formules (I), (II), (III), (IV) et (V) précitées, R désigne un radical alkyle en C₁-C₄, hydroxyalkyle en C₂-C₄, aminoalkyle (C₂-C₄); X désigne un atome d'halogène, de préférence chlore ou brome; R₁ désigne un atome d'hydrogène, d'halogène, un radical alkyle en C₁-C₄; Y désigne le groupement 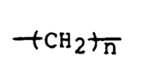
avec n ayant une valeur de 0 à 8, ou bien le groupement 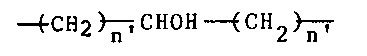
où n' est un nombre entier de 0 à 4.

Le procédé de préparation des composés de formule (V) ou de leurs sels, conforme à l'invention, est essentiellement caractérisé par le fait que :
(1) on condense un hydrocarbure dihalogéné de formule (II) sur une aniline N-substituée (I), dans un solvant polaire et en présence d'un composé basique;
(2) on procède à la nitrosation du composé ainsi formé (III); et
(3) on réduit le dérivé nitrosé (IV) pour obtenir le composé de formule (V).

On utilise dans la première étape du procédé conforme à l'invention, de préférence, des quantités stoechiométriques ou bien un léger excès de l'hydrocarbure dihalogéné de formule (II) par rapport à l'aniline N-substituée de formule (I).

Le solvant polaire est de préférence de l'eau. Le composé basique est utilisé dans des quantités suffisantes pour capter l'acide halohydrique formé et il est de préférence choisi parmi les carbonates alcalins ou alcalino-terreux. La température de réaction varie de préférence entre 50°C et la température de reflux du milieu réactionnel.

La réaction de nitrosation s'effectue essentiellement par l'action d'un mélange nitrite de sodium/acide chlorhydrique dans l'eau, à une température inférieure à 10°C, de préférence comprise entre -2° et +5°C.

La réduction mise en oeuvre dans la troisième étape du procédé s'effectue en milieu éthanolique en présence de chlorure d'ammonium et de zinc à la température de reflux du solvant.

La réduction peut également s'effectuer par voie catalytique dans un solvant constitué d'alcool inférieur en C₁-C₄, d'un mélange eau-alcool, d'acétate d'éthyle ou de tétrahydrofuranne en utilisant comme catalyseur, le nickel, le platine ou le palladium sur charbon.

On peut également procéder à la réduction sous pression d'hydrogène à une température comprise entre 20°C et 100°C et à une pression, de préférence inférieure à 10 bars (1000 k.Pa.).

Les composés de formule (IV) dans laquelle R représente un hydroxyalkyle en C₂-C₄ ou aminoalkyle en C₂-C₄, R₁ et Y ayant les mêmes significations que ci-dessus, sont nouveaux et constituent un autre objet de l'invention.

Un autre objet de l'invention est constitué par le N,N′-bis(β-hydroxyéthyl)N,N′-bis(4′-aminophényl) 1,3-diaminopropane-2-ol et par la N,N′-bis(éthyl)N,N′-bis(4′-amino 3′-méthylphényl)éthylènediamine et leurs sels d'addition avec des acides.

Un autre objet de l'invention est constitué par l'utilisation de l'un ou des deux de ces composés nouveaux ou de leurs sels comme précurseur de colorant d'oxydation de type para dans des compositions tinctoriales d'oxydation pour cheveux. Ces composés conduisent à des teintures stables aux lavages, à la lumière et aux intempéries et possède une bonne innocuité.

Dans les compositions tinctoriales d'oxydation pour cheveux, le composé ci-dessus défini peut être utilisé en association avec des nuanceurs ou coupleurs choisis en particulier parmi les phénols, les méta diphénols, les métaaminophénols, les métaphénylène diamines, les dérivés mono- ou polyhydroxylés du naphtalène et de l'aminonaphtalène, les pyrazolones, les benzomorpholines, les coupleurs hétérocycliques dérivés de la pyridine, le sésamol, et ses dérivés.

Dans les compositions tinctoriales d'oxydation, la concentration du précurseur de colorant défini ci-dessus est comprise généralement entre 0,05 et 12% et de préférence entre 0,1 et 5% en poids. Les coupleurs, lorsqu'ils sont présents, sont utilisés dans des concentrations comprises entre 0,05 et 10% en poids par rapport au poids total de la composition.

Le pH de ces compositions est de préférence alcalin et compris en particulier entre 8 et 11.

Ces compositions contiennent le précurseur de colorant dans un milieu approprié pour la teinture, connu en lui-même, qui peut être un liquide plus ou moins épaissi ou gélifié, une émulsion, une mousse ou toute autre forme appropriée pour la teinture.

Ce milieu est de préférence aqueux et peut être constitué par de l'eau ou un mélange d'eau et d'un solvant organique, tel que l'alcool éthylique, l'alcool propylique ou isopropylique, l'alcool tertiobutylique, l'alcool benzylique ou phényléthylique, l'éthylèneglycol, les polyéthylèneglycols, les éthers monométhylique, monoéthylique ou monobutylique de l'éthylèneglycol, l'acétate du monoéthyléther de l'éthylèneglycol, le propylèneglycol, les monométhyléthers du propylèneglycol et du dipropylèneglycol, le lactate de méthyle.

Ces compositions peuvent contenir tout autre adjuvant habituellement utilisé en teinture des fibres kératiniques, en particulier des agents tensio-actifs anioniques, cationiques, non ioniques, amphotères ou leurs mélanges, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques, etc.

Les compositions conformes à l'invention peuvent également contenir des colorants directs destinés à nuancer la coloration, tels que les dérivés nitrés de la série benzénique, les colorants anthraquinoniques, naphtoquinoniques ou azoïques.

Le procédé de teinture des fibres kératiniques, en particulier des cheveux humains, consiste à appliquer sur ces fibres une composition contenant au moins un précurseur de colorant défini ci-dessus, en une quantité suffisante pour les teindre. De préférence, on mélange au préalable cette composition avec un agent oxydant, tel que le peroxyde d'hydrogène ou un persel, en vue de développer la coloration.

On maintient la composition au contact des fibres pendant une durée de 5 minutes à l heure et on procède ensuite à un rinçage et éventuellement à un lavage, à nouveau un rinçage et un séchage.

Les exemples qui suivent sont destinés à illustrer l'invention sans pour autant présenter un caractère limitatif.

### EXEMPLE DE PREPARATION 1

### Préparation de la N,N′-bis(β-hydroxyéthyl)N,N′-bis (4′-aminophényl)1,3-diaminopropane-2-ol.

### 1ère étape :

### Préparation de la N,N′-bis(β-hydroxyéthyl)N,N′-bis (phényl)1,3-diaminopropane-2-ol.

On chauffe 6 heures, au reflux de l'eau, le mélange constitué par 2 moles (274,4 g) de N-β-hydroxy éthylaniline, de 140 g de carbonate de calcium et 1,2 moles (154,8 g) de 1,3-dichloropropane-2-ol dans l litre d'eau.

Après refroidissement et neutralisation par 50 ml d'acide chlorhydrique concentré, le produit attendu cristallise.

Après essorage, lavage à l'eau, le produit obtenu est recristallisé de l'alcool isopropylique. On obtient 238 g du produit attendu. Il fond à 120°C.

L'analyse du produit obtenu donne les résultats suivants :

| Analyse pour C₁₉H₂₆N₂O₃ | | | | |
|---|---|---|---|---|
| | C% | H% | N% | O% |
| Calculé | 69,06 | 7,93 | 8,48 | 14,53 |
| Trouvé | 69,13 | 7,96 | 8,38 | 14,82 |

### 2ème étape :

### Préparation de la N,N′-bis-(β-hydroxyéthyl) N,N′-bis (4′-nitrosophényl)1,3-diaminopropane-2-ol.

A une solution de 0,5 mole (165,2 g) de N,N′-bis-(β-hydroxyéthyl) N,N′-bis(phényl)1,3-diaminopropane-2-ol préparé à l'étape précédente, de 290 ml d'acide chlorhydrique concentré et de 900 g de glace, on ajoute goutte à goutte entre 0°C et 5°C, une solution de 1,14 moles (78,7 g) de nitrite de sodium dans 150 ml d'eau. L'agitation est maintenue 1 h 30 après la fin de la coulée. Le milieu réactionnel est neutralisé par ajout de 300 ml d'ammoniaque à 20% à 10°C. Le produit obtenu est, après essorage, réempâté dans l'eau. Il peut être utilisé humide pour l'étape suivante.

### 3ème étape :

### Préparation de l'hydrate du tétrachlorhydrate de N,N′-bis-(β-hydroxyéthyl)N,N′-bis[(4′-amino)phényl]1,3-diaminopropane-2-ol.

A 930 ml d'alcool éthylique à 96° additionné de 135 ml d'eau, de 11,9 g de chlorure d'ammonium et 390 g de zinc en poudre fine portés au reflux, on ajoute par portions, 0,58 mole (22,6 g) du dérivé dinitroso préparé à l'étape précédente. Le chauffage est maintenu 1 heure à la fin de l'addition.

Par filtration du milieu réactionnel à chaud, on élimine le zinc et précipite le produit attendu par ajout au filtrat, de 340 ml d'une solution d'acide chlorhydrique dans l'éthanol absolu (7,5 N). Par ajout d'éther éthylique, on obtient après séchage, 234 g de produit attendu.

L'analyse du produit obtenu donne les résultats suivants :

| Analyse pour C₁₅H₂₆N₄O₂Cl₄ | | | | | |
|---|---|---|---|---|---|
| | C% | H% | N% | O% | Cl% |
| Calculé | 43,53 | 6,53 | 10,68 | 12,21 | 27,05 |
| Trouvé | 43,72 | 6,57 | 10,37 | 12,16 | 27,10 |

### EXEMPLE DE PREPARATION 2

### Préparation du dichlorhydrate de N,N′-bis (β-hydroxy éthyl) N,N′-bis(4′-aminophényl)éthylènediamine.

On opère comme dans l'exemple 1, mais dans la première étape, on utilise le 1,2-dibromoéthane à la place du 1,3-dichloropropane-2-ol.

| Analyse pour C₁₈H₂₈N₄O₂Cl₂ | | | | | |
|---|---|---|---|---|---|
| | C% | H% | N% | O% | Cl% |
| Calculé | 53,60 | 7,00 | 13,89 | 7,93 | 17,58 |
| Trouvé | 53,43 | 6,84 | 13,81 | 8,12 | 17,78 |

### EXEMPLE DE PREPARATION 3

### Préparation de l'hydrate de tétrachlorhydrate de N,N′-bis(éthyl)N,N′-bis(4′-amino 3′-méthylphényl) éthylènediamine.

### 1ère étape :

### Préparation du dichlorhydrate de N,N′-bis(éthyl)N,N′-bis (3′-méthylphényl)éthylènediamine.

On chauffe 15 heures au reflux le mélange constitué par 1 mole (135,2 g) de N-éthyl m-toluidine, de 60 g de carbonate de calcium et de 0,55 mole (104 g) de 1,3-dibromoéthane.

Après refroidissement et neutralisation par 60 ml d'acide chlorhydrique concentré, puis extraction à l'acétate d'éthyle, on obtient le produit attendu qui est transformé en dichlorhydrate par une solution d'acide chlorhydrique dans l'éthanol absolu.

Le produit est recristallisé de l'éthanol absolu.

L'analyse du produit obtenu donne les résultats suivants :

| Analyse pour C₂₀H₃₀N₂Cl₂ | | | | |
|---|---|---|---|---|
| | C% | H% | N% | Cl% |
| Calculé | 65,04 | 8,18 | 7,58 | 19,20 |
| Trouvé | 65,14 | 8,17 | 7,52 | 19,12 |

### 2ème étape :

### Préparation de la N,N′-bis(éthyl)N,N′-bis(3′-méthyl 4′-nitrosophényl)éthylènediamine.

A une solution de 0,05 mole (18,5 g) de N,N′-bis(éthyl)N,N′-bis(3′-méthylphényl)éthylènediamine sous forme de dichlorhydrate préparée à l'étape précédente, de 20 ml d'acide chlorhydrique concentré et 105 g de glace, on ajoute goutte à goutte à 0°C une solution de 0,106 mole (7,3 g) de nitrite de sodium dans 17 ml d'eau. L'agitation est maintenue 30 minutes après la fin de la coulée. Le chlorhydrate du produit attendu cristallise.

Par ajout de 15 ml d'ammoniaque à 20% à une suspension du produit obtenu dans 200 ml d'eau, le produit attendu précipite. Recristallisé de 200 ml d'éthanol à 96°, il fond à 157°C.

L'analyse du produit obtenu donne les résultats suivants :

| Analyse pour C₂₀H₂₆N₄O₂ | | | | |
|---|---|---|---|---|
| | C% | H% | N% | O% |
| Calculé | 67,77 | 7,39 | 15,81 | 9,03 |
| Trouvé | 68,03 | 7,42 | 16,06 | 9,16 |

### 3ème étape :

### Préparation de l'hydrate du tétrachlorhydrate de N,N′-bis(éthyl)N,N′-bis(4′-amino 3′-méthylphényl)éthylène diamine.

A 60 ml d'alcool éthylique à 96° additionné de 8 ml d'eau, de 0,8 g de chlorure d'ammonium et de 31 g de zinc en poudre fine portés au reflux, on ajoute par portions 0,039 mole (14,1 g) du dérivé dinitrosé préparé à l'étape précédente, Le chauffage est maintenu 30 minutes après la fin de la coulée.

Par filtration du milieu réactionnel à chaud, on élimine le zinc. Après ajout d'une solution éthanolique d'acide chlorhydrique et évaporation à sec, on obtient le produit attendu.

L'analyse du produit obtenu après purification dans l'éthanol chlorhydrique, donne les résultats suivants :

| Analyse pour C₂₀H₃₆N₄OCl₄ | | | | | |
|---|---|---|---|---|---|
| | C% | H% | N% | O% | Cl% |
| Calculé | 48,99 | 7,40 | 11,43 | 3,26 | 28,92 |
| Trouvé | 48,88 | 7,46 | 11,37 | 3,40 | 28,75 |

### EXEMPLES DE TEINTURE

### EXEMPLE 1

On prépare le mélange tinctorial suivant :

| | |
|---|---|
| - Hydrate de tétrachlorhydrate de N,N′-bis-(β-hydroxyéthyl)N,N′-bis (4′-aminophényl)1,3-diaminopropane-2-ol | 0,535 g |
| - 4-[β-hydroxyéthylamino]2-hydroxytoluène | 0,418 g |
| - Métaaminophénol | 0,112 g |
| - Résorcine | 0,139 g |
| - Dichlorhydrate de 4-(β-hydroxyéthoxy)1,3-phénylènediamine | 0,05 g |
| - Alcool cétylstéarylique vendu sous la dénomination ALFOL C 16/18 par la Société CONDEA | 8,0 g |
| - Cétylstéarylsulfate de sodium vendu sous la dénomination CIRE DE LANETTE E par la Société HENKEL | 0,5 g |
| - Huile de ricin éthoxylée vendue sous la dénomination CEMULSOL B par la Société RHONE POULENC | 1,0 g |
| - Diéthanolamide oléique | 1,5 g |
| - Sel pentasodique de l'acide diéthylènetriamine pentacétique vendu sous la dénomination MASQUOL DTPA par la Société PROTEX | 2,5 g |
| - Ammoniaque 22°Be | 11,0 g |
| - Eau | qsp 100,0 g |
| - pH = 10,1 | |

Au moment de l'emploi, on ajoute 10 g d'eau oxygénée à 20 volumes. Le mélange appliqué 20 minutes à 35°C sur des cheveux naturellement blancs à 90% leur confère, après shampooing et rinçage, une coloration gris légèrement pourpre.

### EXEMPLE 2

On prépare de mélange tinctorial suivant :

| | |
|---|---|
| - Dichlorhydrate de N,N′-bis(β-hydroxyéthyl)N,N′-bis(4′-amino phényl)éthylènediamine | 0,72 g |
| - Hydrate de tétrachlorhydrate de N,N′-bis(éthyl)N,N′-bis(4′-amino 3′-méthylphényl)éthylènediamine | 0,70 g |
| - Dichlorhydrate de 2,4-diaminophénoxy éthanol | 0,128 g |
| - Nonylphénol oxyéthyléné à 4 moles d'oxyde d'éthylène, vendu sous la dénomination CEMULSOL NP 4 par la Société RHONE POULENC | 12,0 g |
| - Nonylphénol oxyéthyléné à 9 moles d'oxyde d'éthylène, vendu sous la dénomination CEMULSOL NP 9 par la Société RHONE POULENC | 15,0 g |
| - Alcool oléique polyglycérolé à 2 moles de glycérol | 1,5 g |
| - Alcool oléique polyglycérolé à 4 moles de glycérol | 1,5 g |
| - Propylèneglycol | 6,0 g |
| - Acide éthylènediamine tétracétique, vendu sous la dénomination TRILON B | 0,12 g |
| - Ammoniaque à 22°Bé | 11,0 g |
| - Eau | qsp 100,0 g |
| - pH = 10,1 | |

Au moment de l'emploi, on ajoute 9 g d'eau oxygénée à 20 volumes. Le mélange appliqué 25 minutes à 30°C sur cheveux permanentés leur confère, après shampooing et rinçage, une coloration bleu légèrement noir.

### EXEMPLE 3

On prépare le mélange tinctorial suivant :

| | |
|---|---|
| - Hydrate de tétrachlorhydrate de N,N'-bis(éthyl)N,N'-bis(4'-amino 3'-méthylphényl)éthylènediamine | 0,834 g |
| - Résorcine | 0,149 g |
| - Dichlorhydrate de 2,4-diamino phénoxyéthanol | 0,399 g |
| - 2-butoxyéthanol | 10,0 g |
| - Alcool cétylstéarylique vendu sous la dénomination ALFOL C 16/18 par la Société CONDEA | 8,0 g |
| - Cétylstéarylsulfate de sodium vendu sous la dénomination CIRE DE LANETTE E par la Société HENKEL | 0,5 g |
| - Huile de ricin éthoxylée vendue sous la dénomination CEMULSOL B par la Société RHONE POULENC | 1,0 g |
| - Diéthanolamide oléique | 1,5 g |
| - Sel pentasodique de l'acide diéthylènetriamine pentacétique, vendu sous la dénomination MASQUOL DTPA par la Société PROTEX | 2,5 g |
| - Ammoniaque à 22°Bé | 11,0 g |
| - Eau | qsp 100,0 g |
| - pH = 9,6 | |

Au moment de l'emploi, on ajoute 10 g d'eau oxygénée à 20 volumes. Le mélange appliqué 20 minutes à 30°C sur cheveux décolorés leur confère, après shampooing et rinçage, une coloration bleu roi.

## Revendications (Revendications pour l'(les) Etat(s) contractant(s) suivant(s): AT, BE, CH, DE, FR, GB, GR, IT, LI, NL, SE)

1. Procédé de préparation d'un composé répondant à la formule : 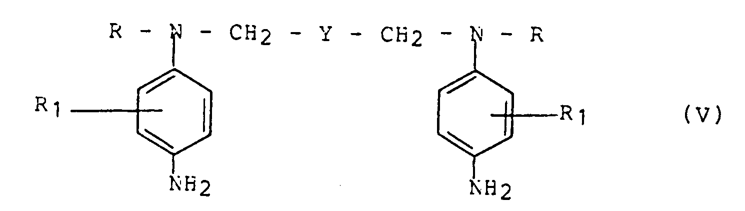 dans laquelle :
R désigne un groupement alkyle en C₁-C₄, hydroxyalkyle en C₂-C₄, aminoalkyle en C₂-C₄;
R₁ désigne un atome d'hydrogène, d'halogène ou un radical alkyle en C₁-C₄;
Y désigne (CH₂)ₙ avec n ayant une valeur de 0 à 8; ou bien 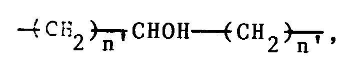 dans laquelle n' est un nombre entier de 0 à 4,
ou de ses sels,
caractérisé par le fait que :
(1) on condense un hydrocarbure dihalogéné de formule :
X - CH₂ - Y - CH₂ - X
sur une aniline N-substituée : 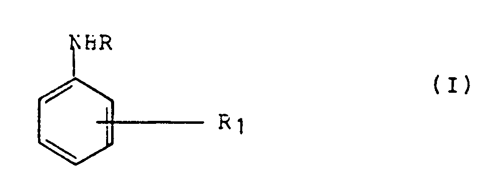 dans un solvant polaire en présence d'un composé basique;
(2) que l'on procède à la nitrosation du composé en résultant, de formule : 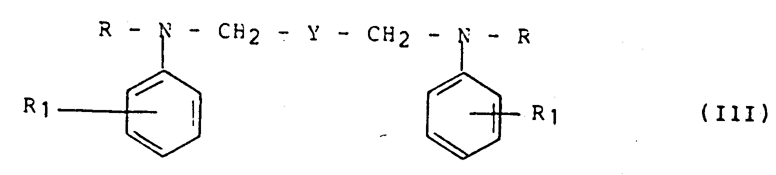
(3) que l'on procède à la réduction du composé ainsi obtenu, de formule : 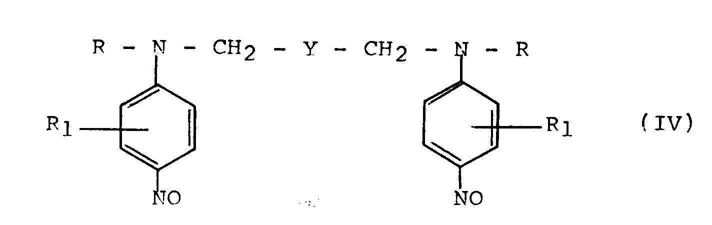 pour préparer le composé de formule (V).

2. Procédé selon la revendication 1, caractérisé par le fait que le composé de formule (II) est utilisé dans des quantités stoechiométriques ou en léger excès par rapport au composé de formule (I).

3. Procédé selon la revendication 1 ou 2, caractérisé par le fait que l'on procède à La nitrosation par action d'un mélange nitrite de sodium/acide chlorhydrique dans l'eau à une température inférieure à 10°.

4. Procédé selon Tes revendications 1 à 3, caractérisé par le fait que l'on effectue la réduction en milieu éthanolique, en présence de chlorure d'ammonium et de zinc à la température de reflux du solvant.

5. Procédé selon les revendications 1 à 3, caractérisé par le fait que l'on procède à une réduction catalytique en milieu solvant constitué d'alcool en C₁-C₄, d'un mélange eau-alcool, d'acétate d'éthyle ou de tétrahydrofuranne.

6. Procédé selon les revendications 1 à 3, caractérisé par le fait que l'on procède à la réduction sous pression d'hydrogène à une température comprise entre 20 et 100°C.

7. Procédé selon les revendications 1 à 6, caractérisé en ce qu'on prépare le N,N'-bis-(β-hydroxyéthyl)N,N'-bis(4'-aminophényl)1,3-diaminopropane-2-ol ou un de ses sels d'addition avec des acides.

8. Procédé selon les revendications 1 à 6, caractérisé en ce qu'on prépare la N,N'-bis(éthyl)N,N'-bis(4'-amino 3'-méthylphényl)éthylènediamine ou un de ses sels d'addition avec des acides.

9. Utilisation du composé préparé selon la revendication 7 ou 8, pour la teinture des fibres kératiniques, en particulier des cheveux.

10. Composition tinctoriale pour fibres kératiniques, en particulier pour cheveux, caractérisée par le fait qu'elle contient, au moins, à titre de précurseur de colorant d'oxydation de type para, le N,N'-bis-(β-hydroxyéthyl)N,N'-bis(4'-aminophényl) 1,3-diaminopropane-2-ol ou la N,N-bis(éthyl)N,N'-bis (4'-amino 3'-méthylphényl)éthylènediamine ou l'un de leurs sels d'addition avec des acides, dans un milieu approprié pour la teinture.

11. Composition selon la revendication 10, caractérisée par le fait qu'elle contient également, au moins, un coupleur.

12. Composition selon la revendication 10 ou 11, caractérisée par le fait que le précurseur de colorant est présent dans des proportions comprises entre 0,05 et 12% en poids par rapport au poids total de la composition.

13. Composition selon la revendication 11, caractérisée par le fait que les coupleurs sont choisis parmi les phénols, les métadiphénols, les métaphénylène diamines, les métaaminophénols; les coupleurs hétérocycliques dérivés de la pyridine, le sésamol et ses dérivés, les dérivés mono- ou polyhydroxylés du naphtalène et de l'aminonaphtalène, les pyrazolones, les benzomorpholines.

14. Composition selon les revendications 10 à 13, caractérisée par le fait que le milieu est aqueux et qu'il est ajusté à un pH alcalin.

15. Procédé de teinture des fibres kératiniques, caractérisé par le fait que l'on applique sur ces fibres au moins une composition telle que définie dans' l'une quelconque des revendications 10 à 14, en présence d'une solution oxydante.

16. Procédé de préparation selon les revendications 1 à 3, conduisant aux composés nitrosés de formule (IV) : 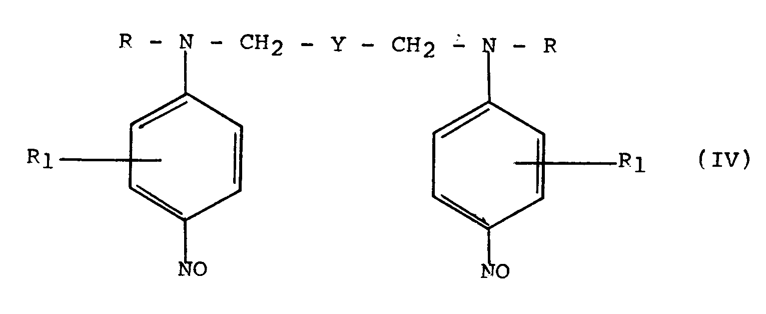 dans laquelle R désigne un radical hydroxyalkyle en C₂-C₄ ou aminoalkyle en C₂-C₄, R₁ et Y ayant les mêmes significations que celles indiquées dans la revendication 1, utilisable comme intermédiaires pour la préparation des composés de formule (V) selon les revendications 1, 4 et 5.

## Revendications (Revendications pour l'(les) Etat(s) contractant(s) suivant(s): ES)

1. Procédé de préparation d'un composé répondant à la formule : 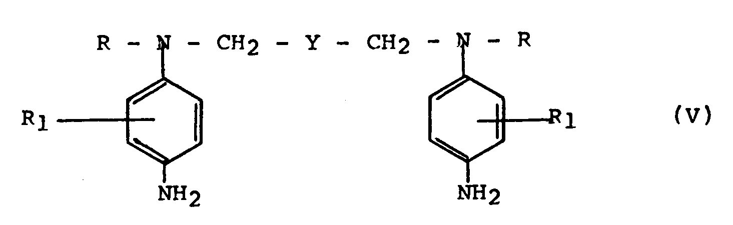 dans laquelle :
R désigne un groupement alkyle en C₁-C₄, hydroxyalkyle en C₂-C₄, aminoalkyle en C₂-C₄;
R₁ désigne un atome d'hydrogène, d'halogène ou un radical alkyle en C₁-C₄;
Y désigne (CH₂)ₙ avec n ayant une valeur de 0 à 8, ou bien 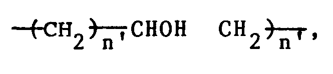 dans laquelle n' est un nombre entier de 0 à 4,
ou de ses sels,
caractérisé par le fait que :
(1) on condense un hydrocarbure dihalogéné de formule :
X - CH₂ - Y - CH₂ - X
sur une aniline N-substituée : 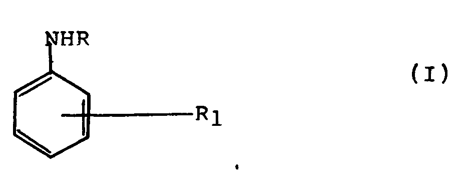 dans un solvant polaire en présence d'un composé basique;
(2) que l'on procède à la nitrosation du composé en résultant, de formule : 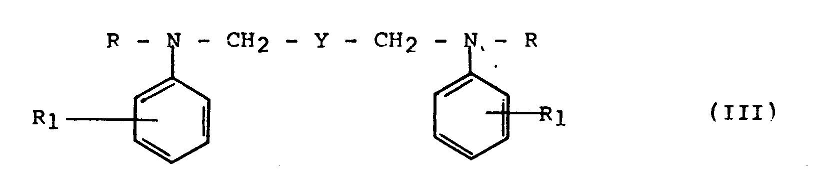
(3) que l'on procède à la réduction du composé ainsi obtenu, de formule : 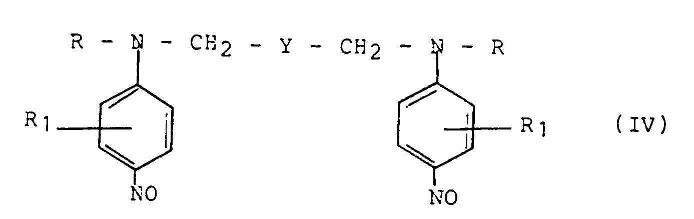 pour préparer le composé de formule (V).

2. Procédé selon la revendication 1, caractérisé par le fait que le composé de formule (II) est utilisé dans des quantités stoechiométriques ou en léger excès par rapport au composé de formule (I).

3. Procédé selon la revendication 1 ou 2, caractérisé par le fait que l'on procède à la nitrosation par action d'un mélange nitrite de sodium/acide chlorhydrique dans l'eau à une température inférieure à 10°.

4. Procédé selon les revendications 1 à 3, caractérisé par le fait que l'on effectue la réduction en milieu éthanolique, en présence de chlorure d'ammonium et de zinc à la température de reflux du solvant.

5. Procédé selon les revendications 1 à 3, caractérisé par le fait que l'on procède à une réduction catalytique en milieu solvant constitué d'alcool en C₁-C₄, d'un mélange eau-alcool, d'acétate d'éthyle ou de tétrahydrofuranne.

6. Procédé selon les revendications 1 à 3, caractérisé par le fait que l'on procède à la réduction sous pression d'hydrogène à une température comprise entre 20 et 100°C.

7. N,N'-bis-(β-hydroxyéthyl)N,N'-bis(4'-amino phényl)1,3-diaminopropane-2-ol ou un de ses sels d'addition avec des acides.

8. N,N'-bis(éthyl)N,N'-bis(4'-amino 3'-méthyl phényl)éthylènediamine ou un de ses sels d'addition avec des acides.

9. Utilisation du composé selon la revendication 7 ou 8, pour la teinture des fibres kératiniques, en particulier des cheveux.

10. Composition tinctoriale pour fibres kératiniques, en particulier pour cheveux, caractérisée par le fait qu'elle contient, au moins, à titre de précurseur de colorant d'oxydation de type para, le N,N'-bis-(β-hydroxyéthyl)N,N'-bis(4'-aminophényl) 1,3-diaminopropane-2-ol ou la N,N-bis(éthyl)N,N'-bis (4'-amino 3'-méthylphényl)éthylènediamine ou l'un de leurs sels d'addition avec des acides, dans un milieu approprié pour la teinture.

11. Composition selon la revendication 10, caractérisée par le fait qu'elle contient également, au moins, un coupleur.

12. Composition selon la revendication 10 ou 11, caractérisée par le fait que le précurseur de colorant est présent dans des proportions comprises entre 0,05 et 12% en poids par rapport au poids total de la composition.

13. Composition selon la revendication 11, caractérisée par le fait que les coupleurs sont choisis parmi les phénols, les métadiphénols, les métaphénylène diamines, les métaaminophénols; les coupleurs hétérocycliques dérivés de la pyridine, le sésamol et ses dérivés, les dérivés mono- ou polyhydroxylés du naphtalène et de l'aminonaphtalène, les pyrazolones, les benzomorpholines.

14. Composition selon les revendications 10 à 13, caractérisée par le fait que le milieu est aqueux et qu'il est ajusté à un pH alcalin.

15. Procédé de teinture des fibres kératiniques, caractérisé par le fait que l'on applique sur ces fibres au moins une composition telle que définie dans l'une quelconque des revendications 10 à 14, en présence d'une solution oxydante.

16. Composés utilisables comme intermédiaires dans le procédé selon la revendication 1, caractérisés par le fait qu'ils répondent à la formule : 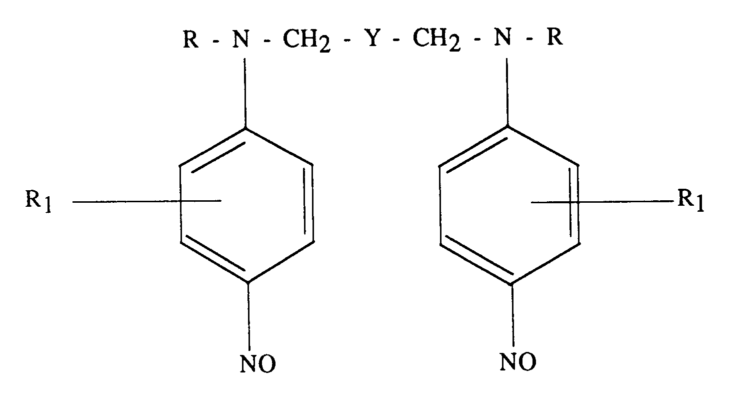 dans laquelle R désigne un radical hydroxyalkyle en C₂-C₄ ou aminoalkyle en C₂-C₄, R₁ désigne un atome d'hydrogène ou d'halogène et Y a la même signification que celle indiquée dans la revendication 1.

## Claims (Claims for the following Contracting State(s): AT, BE, CH, DE, FR, GB, GR, IT, LI, NL, SE)

1. Process for preparing a compound corresponding to the formula: 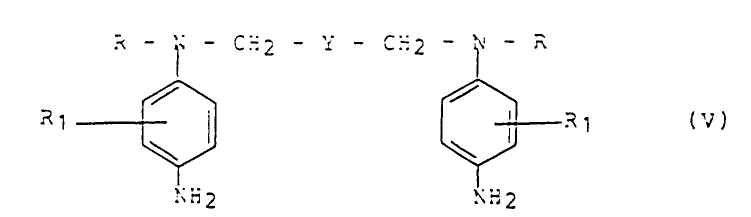 in which:
R denotes a C₁-C₄ alkyl, C₂-C₄ hydroxyalkyl or C₂-C₄ aminoalkyl group;
R₁ denotes a hydrogen or halogen atom or a C₁-C₄ alkyl radical;
Y denotes (CH₂)ₙ with n having a value from 0 to 8, or else -(CH₂)_{n'}-CHOH-(CH₂)_{n'}-, in which n' is an integer from 0 to 4,
or its salts,
characterized in that:
(1) a dihalogenated hydrocarbon of formula:
X-CH₂-Y-CH₂-X
is condensed with an N-substituted aniline: 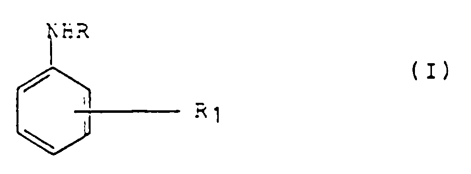 in a polar solvent in the presence of a basic compound;
(2) the compound resulting therefrom, of formula: 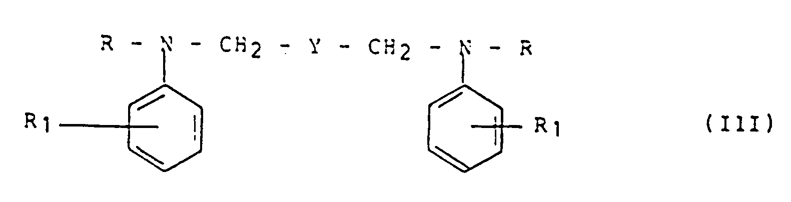 is nitrosated;
(3) the compound thus obtained, of formula: 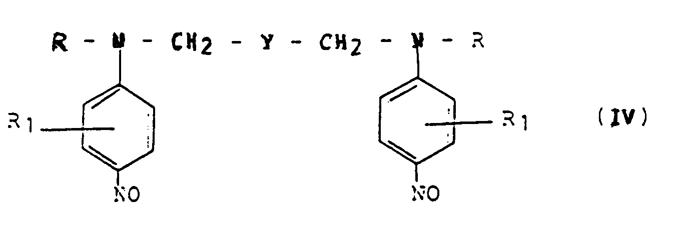 is reduced to prepare the compound of formula (V).

2. Process according to Claim 1, characterized in that the compound of formula (II) is employed in stoichiometric quantities or in a slight excess relative to the compound of formula (I).

3. Process according to Claim 1 or 2, characterized in that the nitrosation is carried out by reaction with a sodium nitrite/hydrochloric acid mixture in water at a temperature below 10°.

4. Process according to Claims 1 to 3, characterized in that the reduction is carried out in an ethanolic medium in the presence of ammonium chloride and zinc at the reflux temperature of the solvent.

5. Process according to Claims 1 to 3, characterized in that a catalytic reduction is carried out in a solvent medium consisting of C₁-C₄ alcohol, of a mixture of water and alcohol, of ethyl acetate or of tetrahydrofuran.

6. Process according to Claims 1 to 3, characterized in that the reduction is carried out under hydrogen pressure at a temperature of between 20 and 100°C.

7. N,N'-Bis(β-hydroxyethyl)-N,N'-bis(4'-aminophenyl)-1,3-diamino-2-propanol or one of its addition salts with acids.

8. N,N'-Bis(ethyl)-N,N'-bis(4'-amino-3'-methylphenyl)ethylenediamine or one of its addition salts with acids.

9. Use of the compound according to Claim 7 or 8 for dyeing keratinous fibres, in particular hair.

10. Dye composition for keratinous fibres, in particular for hair, characterized in that it contains, at least, as an oxidation dye precursor of para type, N,N'-bis(β-hydroxyethyl)-N,N'-bis(4'-aminophenyl)-1,3-diamino-2-propanol or N,N'-bis(ethyl)-N,N'-bis(4'-amino-3'-methylphenyl)ethylenediamine or one of their addition salts with acids, in a suitable medium for dyeing.

11. Composition according to Claim 10, characterized in that it also contains, at least, coupler.

12. Composition according to Claim 10 or 11, characterized in that the dye precursor is present in proportions of between 0.05 and 12 % by weight relative to the total weight of the composition.

13. Composition according to Claim 11, characterized in that the couplers are chosen from phenols, meta-diphenols, meta-phenylenediamines, meta-aminophenols, heterocyclic couplers derived from pyridine, sesamol and its derivatives, mono- or polyhydroxylated derivatives of naphthalene and of aminonaphthalene, pyrazolones and benzomorpholines.

14. Composition according to Claims 10 to 13, characterized in that the medium is aqueous and is adjusted to an alkaline pH.

15. Process for dyeing keratinous fibres, characterized in that at least one composition as defined in any one of Claims 10 to 14 is applied to these fibres in the presence of an oxidizing solution.

16. Compounds which can be used as intermediates in the process according to Claim 1, characterized in that they correspond to the formula 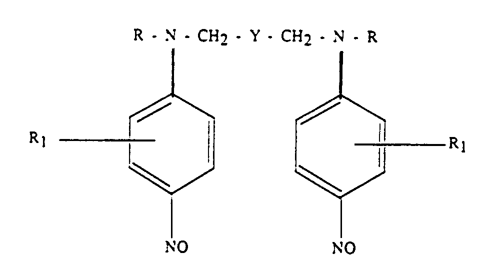 in which R denotes a C₂-C₄ hydroxyalkyl or C₂-C₄ aminoalkyl radical, R₁ denotes a hydrogen or halogen atom, and Y has the same meaning as that shown in Claim 1.

## Claims (Claims for the following Contracting State(s): ES)

1. Process for preparing a compound corresponding to the formula: 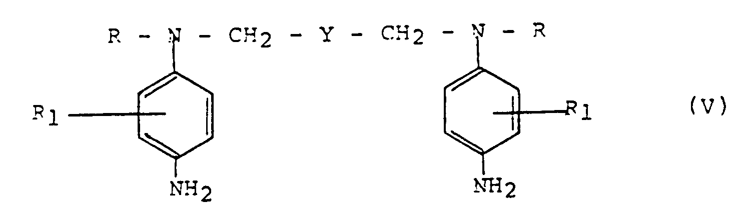 in which:
R denotes a C₁-C₄ alkyl, C₂-C₄ hydroxyalkyl or C₂-C₄ aminoalkyl group;
R₁ denotes a hydrogen or halogen atom or a C₁-C₄ alkyl radical;
Y denotes (CH₂)ₙ with n having a value from 0 to 8, or else -(CH₂)_{n'}-CHOH-(CH₂)_{n'}-, in which n' is an integer from 0 to 4,
or its salts,
characterized in that:
(1) a dihalogenated hydrocarbon of formula:
X-CH₂-Y-CH₂-X
is condensed with an N-substituted aniline: 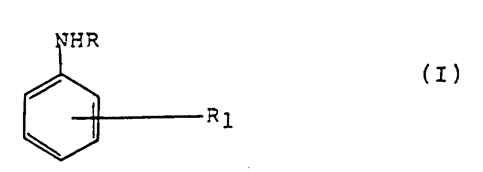 in a polar solvent in the presence of a basic compound;
(2) the compound resulting therefrom, of formula: 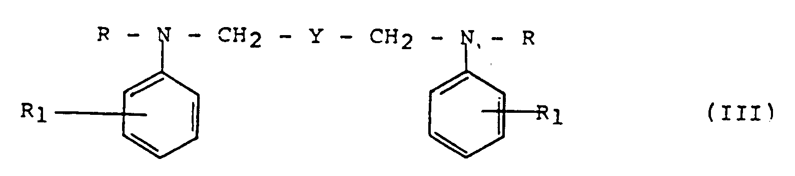 is nitrosated;
(3) the compound thus obtained, of formula: 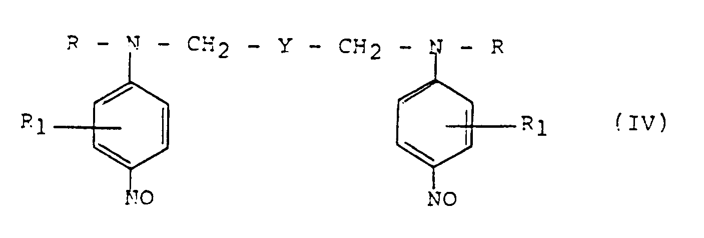 is reduced to prepare the compound of formula (V).

2. Process according to Claim 1, characterized in that the compound of formula (II) is employed in stoichiometric quantities or in a slight excess relative to the compound of formula (I).

3. Process according to Claim 1 or 2, characterized in that the nitrosation is carried out by reaction with a sodium nitrite/hydrochloric acid mixture in water at a temperature below 10°.

4. Process according to Claims 1 to 3, characterized in that the reduction is carried out in an ethanolic medium in the presence of ammonium chloride and zinc at the reflux temperature of the solvent.

5. Process according to Claims 1 to 3, characterized in that a catalytic reduction is carried out in a solvent medium consisting of C₁-C₄ alcohol, of a mixture of water and alcohol, of ethyl acetate or of tetrahydrofuran.

6. Process according to Claims 1 to 3, characterized in that the reduction is carried out under hydrogen pressure at a temperature of between 20 and 100°C.

7. Process according to Claims 1 to 6, characterized in that N,N'-bis(β-hydroxyethyl)-N,N'-bis(4'-aminophenyl)-1,3-diamino-2-propanol or one of its addition salts with acids is prepared.

8. Process according to Claims 1 to 6, characterized in that N,N'-bis(ethyl)-N,N'-bis(4'-amino-3'-methylphenyl)ethylenediamine or one of its addition salts with acids is prepared.

9. Use of the compound according to Claim 7 or 8 for dyeing keratinous fibres, in particular hair.

10. Dye composition for keratinous fibres, in particular for hair, characterized in that it contains, at least, as an oxidation dye precursor of para type, N,N'-bis(β-hydroxyethyl)-N,N'-bis(4'-aminophenyl)-1,3-diamino-2-propanol or N,N'-bis(ethyl)-N,N'-bis(4'-amino-3'-methylphenyl)ethylenediamine or one of their addition salts with acids, in a suitable medium for dyeing.

11. Composition according to Claim 10, characterized in that it also contains, at least, coupler.

12. Composition according to Claim 10 or 11, characterized in that the dye precursor is present in proportions of between 0.05 and 12 % by weight relative to the total weight of the composition.

13. Composition according to Claim 11, characterized in that the couplers are chosen from phenols, meta-diphenols, meta-phenylenediamines, meta-aminophenols, heterocyclic couplers derived from pyridine, sesamol and its derivatives, mono- or polyhydroxylated derivatives of naphthalene and of aminonaphthalene, pyrazolones and benzomorpholines.

14. Composition according to Claims 10 to 13, characterized in that the medium is aqueous and is adjusted to an alkaline pH.

15. Process for dyeing keratinous fibres, characterized in that at least one composition as defined in any one of Claims 10 to 14 is applied to these fibres in the presence of an oxidizing solution.

16. Preparation process according to Claims 1 to 3, leading to the nitrosated compounds of formula (IV): 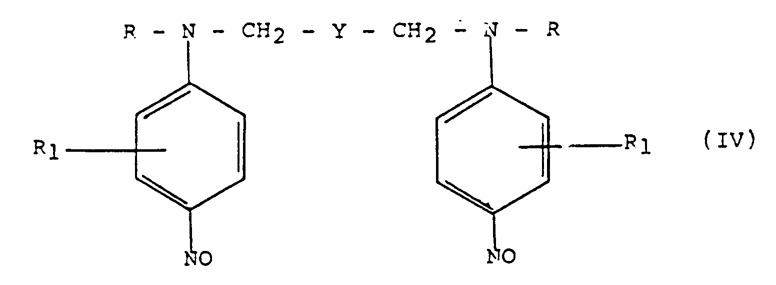 in which R denotes a C₂-C₄ hydroxyalkyl or C₂-C₄ aminoalkyl radical, R₁ and Y having the same meanings as those given in Claim 1, useful as intermediates for the preparation of the compounds of formula (V) according to Claims 1, 4 and 5.

## Patentansprüche (Patentansprüche für folgende(n) Vertragsstaat(en): AT, BE, CH, DE, FR, GB, GR, IT, LI, NL, SE)

1. Verfahren zur Herstellung einer Verbindung der Formel: 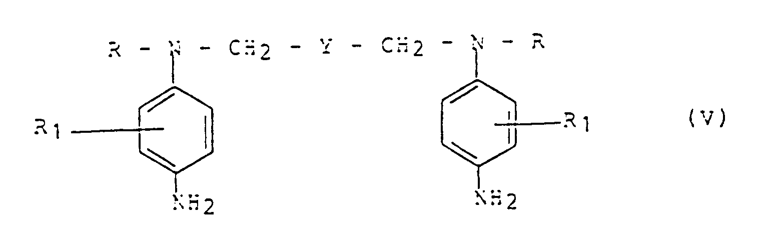 worin:
R einen C₁₋₄-Alkyl-, C₂₋₄-Hydroxyalkyl-, C₂₋₄-Aminoalkylrest,
R₁ ein Wasserstoff-, Halogenatom oder einen C₁₋₄-Alkylrest,
Y -(CH₂)-ₙ mit einem Wert für n von 0 bis 8 oder auch die Gruppe -(CH₂)-_{n'}-CHOH-(CH₂)_{n'}- bedeuten, worin n' eine ganze Zahl von 0 bis 4 ist,
oder von ihren Salzen,
dadurch **gekennzeichnet,** daß man:
(1) einen dihalogenierten Kohlenwasserstoff der Formel:
X - CH₂ - Y - CH₂ - X (II)
mit einem N-substituierten Anilin: 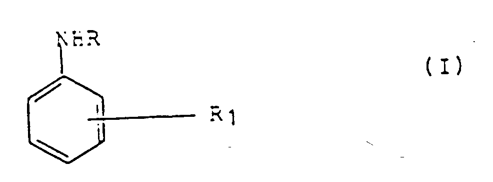 in einem polaren Lösungsmittel in Gegenwart einer basischen Verbindung kondensiert,
(2) die entstandene Verbindung der Formel: 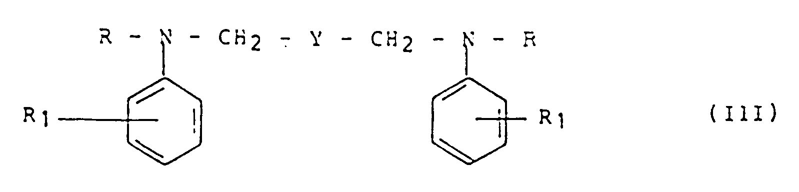 nitrosiert,
(3) die so erhaltene Verbindung der Formel: 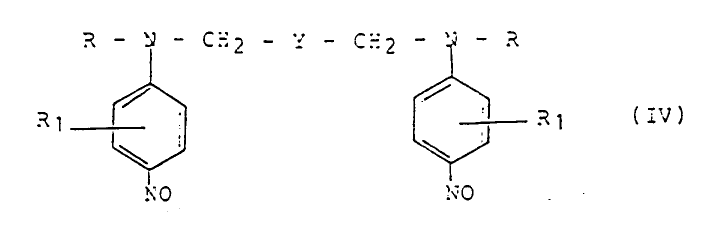 reduziert, um die Verbindung der Formel (V) herzustellen.

2. Verfahren gemäß Anspruch 1,
dadurch **gekennzeichnet,** daß
die Verbindung der Formel (II) in stöchiometrischen Mengen oder in leichtem Überschuß eingesetzt wird, bezogen auf die Verbindung der Formel (I).

3. Verfahren gemäß Anspruch 1 oder 2,
dadurch **gekennzeichnet,** daß
man mit einer Mischung aus Natriumnitrit/Salzsäure in Wasser bei einer Temperatur unterhalb 10°C nitrosiert.

4. Verfahren gemäß Anspruch 1 bis 3,
dadurch **gekennzeichnet,** daß
man in ethanolischem Medium in Gegenwart von Ammoniumchlorid und Zink bei der Rückflußtemperatur des Lösungsmittels reduziert.

5. Verfahren gemäß Anspruch 1 bis 3,
dadurch **gekennzeichnet,** daß
man eine katalytische Reduktion in einem Lösungsmittelmedium aus C₁₋₄-Alkohol, einer Mischung Wasser-Alkohol, aus Ethylacetat oder Tetrahydrofuran durchführt.

6. Verfahren gemäß Ansprüchen 1 bis 3,
dadurch **gekennzeichnet,** daß
man die Reduktion unter Wasserstoffdruck bei einer Temperatur von 20 bis 100°C durchführt.

7. N,N'-Bis(β-hydroxyethyl)N,N'-bis(4'-aminophenyl)1,3-diaminopropan-2-ol oder eines seiner Säureadditionssalze.

8. N,N'-Bis(ethyl)N,N'-bis(4'-amino-3'-methylphenyl)ethylendiamin oder eines seiner Säureadditionssalze.

9. Verwendung der Verbindung gemäß Anspruch 7 oder 8 zur Färbung keratinischer Fasern, insbesondere der Haare.

10. Färbezusammensetzung für keratinische Fasern, insbesondere für Haare, dadurch **gekennzeichnet,** daß
sie mindestens als Oxidationsfarbstoffvorstufe vom para-Typ N,N'-Bis(β-Hydroxyethyl)N,N'-bis(4'-aminophenyl)1,3-diaminopropan-2-ol oder N,N'-Bis(ethyl)N,N'-bis(4'-amino-3'-methylphenyl)ethylendiamin oder eines von deren Säureadditionssalzen in einem zur Färbung geeigneten Medium enthält.

11. Zusammensetzung gemäß Anspruch 10,
dadurch **gekennzeichnet,** daß
sie auch mindestens einen Kuppler enthält.

12. Zusammensetzung gemäß Anspruch 10 oder 11,
dadurch **gekennzeichnet,** daß
die Farbstoffvorstufe in Mengenverhältnissen von 0,05 bis 12 Gew.% vorliegt, bezogen auf Gesamtgewicht der Zusammensetzung.

13. Zusammensetzung gemäß Anspruch 11,
dadurch **gekennzeichnet,** daß
die Kuppler ausgewählt sind aus Phenolen, m-Diphenolen, m-Phenylendiaminen, m-Aminophenolen, von Pyridin abgeleiteten heterozyklischen Kupplern, Sesamol und seinen Derivaten, mono- oder polyhydroxylierten Derivaten von Naphthalin und Aminonaphthalin, Pyrazolonen, Benzomorpholinen.

14. Zusammensetzung gemäß Anspruch 10 bis 13,
dadurch **gekennzeichnet,** daß
das Medium wässrig und auf einen alkalischen pH eingestellt ist.

15. Verfahren zur Färbung keratinischer Fasern,
dadurch **gekennzeichnet,** daß
man auf die Fasern mindestens eine in jedem der Ansprüche 10 bis 14 definierte Zusammensetzung in Gegenwart einer oxidierenden Lösung aufbringt.

16. Verbindungen zur Verwendung als Zwischenprodukte im Verfahren gemäß Anspruch 1,
dadurch **gekennzeichnet,** daß
sie die Formel aufweisen: 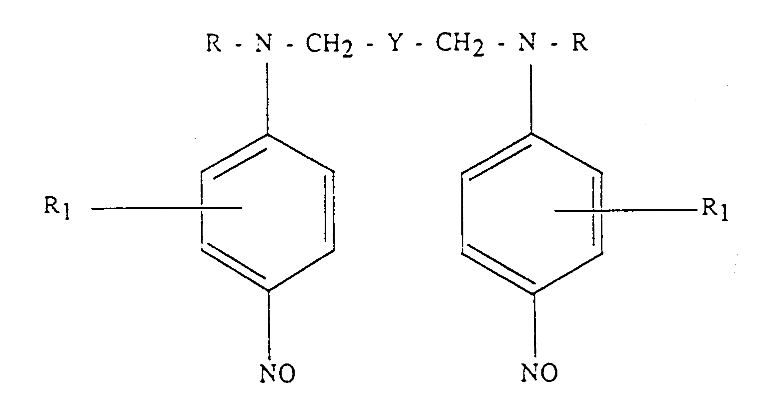 worin R einen C₂₋₄-Hydroxyalkyl- oder C₂₋₄-Aminoalkylrest, R₁ ein Wasserstoff- oder Halogenatom bedeuten und Y dieselbe Bedeutung wie die in Anspruch 1 angegebene hat.

## Patentansprüche (Patentansprüche für folgende(n) Vertragsstaat(en): ES)

1. Verfahren zur Herstellung einer Verbindung der Formel: 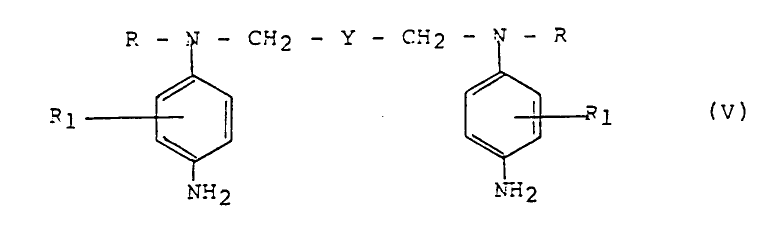 worin:
R einen C₁₋₄-Alkyl-, C₂₋₄-Hydroxyalkyl-, C₂₋₄-Aminoalkylrest,
R₁ ein Wasserstoff-, Halogenatom oder einen C₁₋₄-Alkylrest,
Y -(CH₂)-ₙ mit einem Wert für n von 0 bis 8 oder auch die Gruppe -(CH₂)-_{n'}-CHOH-(CH₂)_{n'}- bedeuten, worin n' eine ganze Zahl von 0 bis 4 ist
oder von ihren Salzen,
dadurch **gekennzeichnet,** daß man:
(1) einen dihalogenierten Kohlenwasserstoff der Formel:
X - CH₂ - Y - CH₂ - X (II)
mit einem N-substituierten Anilin: 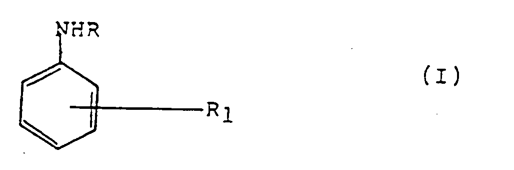 in einem polaren Lösungsmittel in Gegenwart einer basischen Verbindung kondensiert,
(2) die entstandene Verbindung der Formel: 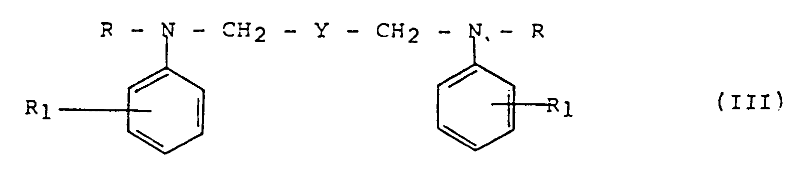 nitrosiert,
(3) die so erhaltene Verbindung der Formel: 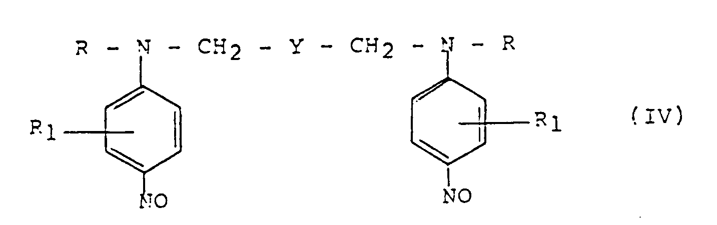 reduziert, um die Verbindung der Formel (V) herzustellen.

2. Verfahren gemäß Anspruch 1,
dadurch **gekennzeichnet,** daß
die Verbindung der Formel (II) in stöchiometrischen Mengen oder in leichtem Überschuß eingesetzt wird, bezogen auf die Verbindung der Formel (I).

3. Verfahren gemäß Anspruch 1 oder 2,
dadurch **gekennzeichnet,** daß
man mit einer Mischung aus Natriumnitrit/Salzsäure in Wasser bei einer Temperatur unterhalb 10°C nitrosiert.

4. Verfahren gemäß Anspruch 1 bis 3,
dadurch **gekennzeichnet,** daß
man in ethanolischem Medium in Gegenwart von Ammoniumchlorid und Zink bei der Rückflußtemperatur des Lösungsmittels reduziert.

5. Verfahren gemäß Anspruch 1 bis 3,
dadurch **gekennzeichnet,** daß
man eine katalytische Reduktion in einem Lösungsmittelmedium aus C₁₋₄-Alkohol, einer Mischung Wasser-Alkohol, aus Ethylacetat oder Tetrahydrofuran durchführt.

6. Verfahren gemäß Ansprüchen 1 bis 3,
dadurch **gekennzeichnet,** daß
man die Reduktion unter Wasserstoffdruck bei einer Temperatur von 20 bis 100°C durchführt.

7. Verfahren gemäß Ansprüchen 1 bis 6,
dadurch **gekennzeichnet,** daß
man N,N'-Bis(β-hydroxyethyl)N,N'-bis(4'-aminophenyl)1,3-diaminopropan-2-ol oder eines seiner Säureadditionssalze herstellt.

8. Verfahren gemäß Ansprüchen 1 bis 6,
dadurch **gekennzeichnet,** daß
man N,N'-Bis(ethyl)N,N'-bis(4'-amino-3'-methylphenyl)ethylendiamin oder eines seiner Säureadditionssalze herstellt.

9. Verwendung der Verbindung gemäß Anspruch 7 oder 8 zur Färbung keratinischer Fasern, insbesondere der Haare.

10. Färbezusammensetzung für keratinische Fasern, insbesondere für Haare, dadurch **gekennzeichnet,** daß
sie mindestens als Oxidationsfarbstoffvorstufe vom para-Typ N,N'-Bis(β-Hydroxyethyl)N,N'-bis(4'-aminophenyl)1,3-diaminopropan-2-ol oder N,N'-Bis(ethyl)N,N'-bis(4'-amino-3'-methylphenyl)ethylendiamin oder eines von deren Säureadditionssalzen in einem zur Färbung geeigneten Medium enthält.

11. Zusammensetzung gemäß Anspruch 10,
dadurch **gekennzeichnet,** daß
sie auch mindestens einen Kuppler enthält.

12. Zusammensetzung gemäß Anspruch 10 oder 11,
dadurch **gekennzeichnet,** daß
die Farbstoffvorstufe in Mengenverhältnissen von 0,05 bis 12 Gew.% vorliegt, bezogen auf Gesamtgewicht der Zusammensetzung.

13. Zusammensetzung gemäß Anspruch 11,
dadurch **gekennzeichnet,** daß
die Kuppler ausgewählt sind aus Phenolen, m-Diphenolen, m-Phenylendiaminen, m-Aminophenolen, von Pyridin abgeleiteten heterozyklischen Kupplern, Sesamol und seinen Derivaten, mono- oder polyhydroxylierten Derivaten von Naphthalin und Aminonaphthalin, Pyrazolonen, Benzomorpholinen.

14. Zusammensetzung gemäß Anspruch 10 bis 13,
dadurch **gekennzeichnet,** daß
das Medium wässrig und auf einen alkalischen pH eingestellt ist.

15. Verfahren zur Färbung keratinischer Fasern,
dadurch **gekennzeichnet,** daß
man auf die Fasern mindestens eine in jedem der Ansprüche 10 bis 14 definierte Zusammensetzung in Gegenwart einer oxidierenden Lösung aufbringt.

16. Herstellungsverfahren gemäß Ansprüchen 1 bis 3, welches zu den nitrosierten Verbindungen der Formel (IV) führt: 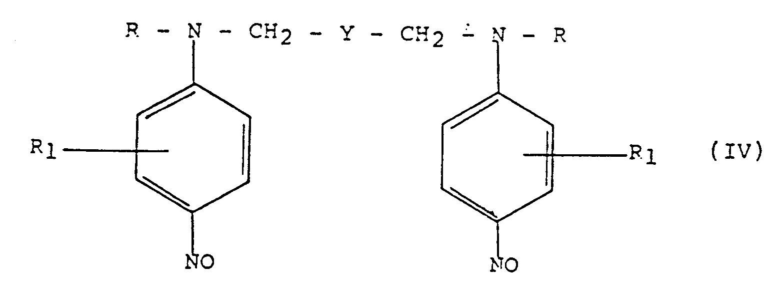 worin R einen C₂₋₄-Hydroxyalkyl- oder C₂₋₄-Aminoalkylrest bedeutet und R₁ und Y dieselben Bedeutungen wie die in Anspruch 1 angegebenen haben, zur Verwendung als Zwischenprodukte zur Herstellung der Verbindungen der Formel (V) gemäß den Ansprüchen 1, 4 und 5.
